# EUROPÄISCHE PATENTANMELDUNG

(11) **EP 1 396 722 A1**
(43) Veröffentlichungstag der Anmeldung: **10.03.2004**
(21) Anmeldenummer: 02020178.6
(22) Anmeldetag: 09.09.2002
(51) Int. Cl.: G01N 33/24

(54) **Messgerät zur Bestimmung der Bodenwasserspannung und der Bodenwasserleitfähigkeit**

(71) Anmelder: UMS Umweltanalytische Mess-Systeme GmbH, 81379 München (DE)
(72) Erfinder: von Unold, Georg, 81379 München (DE)
(74) Vertreter: Finck, Dieter, Dr.Ing.

(57) **Zusammenfassung**

Ein Messgerät (1 ) zur Bestimmung der Bodenwasserspannung und der Bodenwasserleitfähigkeit weist ein Gehäuse (2) mit einer semipermeablen Membran (Keramik 10), eine innerhalb des Gehäuses (2) angeordnete Messstrecke (14), die über die semipermeable Membran (10) mit dem Bodenwasser in Kontakt steht, und eine steuerbare, mit der Messstrecke (14) in Verbindung stehende Pumpe (Schlauchpumpe 16) auf, mittels der Bodenwasser förderbar ist.

Dieses Messgerät gewinnt an Vielseitigkeit und eröffnet neue Möglichkeiten der Bodenuntersuchung.

## Beschreibung

Die Erfindung bezieht sich auf ein Messgerät zur Bestimmung der Bodenwasserspannung mit einem Gehäuse mit einer semipermeablen Membran und einer innerhalb des Gehäuses angeordneten Messstrecke, die über die semipermeable Membran mit dem Bodenwasser in Kontakt steht. Ein solches Messgerät ist aus der DE 196 16 391 C2 bekannt.

Bei dem bekannten Messgerät wird das durch die Membran in das Innere des Gehäuses eingetretene Bodenwasser durch eine Messstrecke geleitet, in der die interessierenden physikalischen Eigenschaften erfasst werden. Bei einem solchen Messgerät handelt es sich um ein passives Gerät, dessen Funktion einzig davon abhängt, ob sich im Gehäuse genügend Bodenwasser befindet oder nicht. Insbesondere ist es nicht möglich, dem Boden mittels des Geräts Bodenwasser zu entnehmen oder dem Boden geregelt zuzuführen, zu infiltrieren.

Durch die Erfindung wird ein solches Messgerät zur Verfügung gestellt: Es enthält eine steuerbare, mit der Messstrecke in Verbindung stehende Pumpe, mittels der das Bodenwasser förderbar ist. Als Pumpe eignet sich grundsätzlich jede Pumpenart, die im Stillstand dicht abschließt, also beispielsweise eine Kolbenoder Membranpumpe, oder die durch ein nachgeschaltetes Ventil abgedichtet wird. Besonders bevorzugt ist eine Schlauchpumpe, weil sie sehr einfach aufgebaut ist und auch nach längerer Betriebszeit dicht bleibt.

Das erfindungsgemäße Messgerät ist vorzugsweise als Tensiometer ausgebildet, das heißt, an der Messstrecke ist ein Druckaufnehmer angeordnet. Bei Tensiometern überträgt sich die Bodenwasserspannung über den kapillaren Verbund auf das Wasser im Tensiometer und ist als atmosphärischer Unterdruck messbar. Dieser kann mit Hilfe des z.B. elektronischen Drucksensors gemessen und zur Anzeige gebracht oder mit Datenloggern aufgezeichnet werden.

Ordnet man nach der im Anspruch 3 beschriebenen bevorzugten Ausführungsform an der Messstrecke einen die Gegenwart von Wasser in derselben erfassenden Sensor an, z. B. eine Infrarot-Messstrecke oder einen Wärmeverteilungssensor, lässt sich die Pumpe so steuern, dass sie nur anspricht, wenn Wasser vorhanden ist.

Die Bodenwasserspannung ist das Maß für die Summe der Haltekräfte für das Wasser im Boden. Sie ist entscheidend für pflanzenphysiologische Untersuchungen, da Pflanzen dieses Potential überwinden müssen, um dem Boden Wasser zu entnehmen. Zugleich ist die Kenntnis der Bodenwasserspannung nötig für Wassertransport- oder Wasserhaushaltsstudien, da Wasser immer von Orten höheren Potentials zu Orten niedrigeren Potentials fließt. Andere übliche Begriffe sind das "Matrix-Potential" oder "Wasserrückhaltevermögen" des Bodens.

Vorzugsweise ist das erfindungsgemäße Messgerät mit einer Steigleitung versehen (Anspruch 4), mittels der das angesammelte Bodenwasser aus dem Gehäuse heraus in den Schacht oder zur Bodenoberfläche förderbar ist, wobei die Pumpe vorzugsweise als direkt auf die Steigleitung wirkende Schlauchpumpe ausgebildet ist (Anspruch 5). Soll das Bodenwasser nicht an die Bodenoberfläche gepumpt, sondern wieder in den Boden zurückgeleitet werden, wird die Steigleitung an eine in der Gehäusewandung vorgesehene Membran angeschlossen, so dass das Bodenwasser direkt in den Boden rückgeleitet werden kann.

Die Steigleitung ist vorzugsweise an ein Sammelgefäß angeschlossen, das, wie z. B. aus der DE 196 16 391 C2 bekannten, vorzugsweise in das Gehäuse integriert ist Anspruch 6). Auf diese Weise lässt sich ein völlig wartungsfreies Messgerät zur Bestimmung der Bodenwasserspannung schaffen.

Das erfindungsgemäße Tensiometer kann, ebenso wie bekannte Tensiometer, beim Einsetzen in den Boden mit Wasser gefüllt werden. Es kann aber auch trocken eingesetzt werden, wobei es einsatzfähig wird, sobald die Messstrecke mit eingedrungenem Bodenwasser gefüllt ist.

Die Pumpe (Peristaltikpumpe) entfernt zeitlich versetzt die in der Messkammer störende, durch den Sensor erfasste Luft durch Absaugen der Luft aus der Messkammer am höchsten Punkt. Durch das Absaugen entsteht ein Unterdruck, wodurch bis zum Dampfpunkt Bodenwasser in die Messkammer gesaugt wird. Der Messwert kann aktiv stabilisiert werden durch eine Gradienten-, Drehzahl- und Drehrichtungssteuerung der Schlauchpumpe. Luftblasen in Tensiometern nach dem Stand der Technik stören dadurch den Messwert nicht mehr, da sich das Tensiometer selbst befüllt, indem die Pumpe die Luftblasen vom höchsten Punkt der Messkammer absaugt, wodurch bis zum Erreichen der Gleichgewichtswasserspannung Bodenwasser über die poröse Keramik in die Messkammer fließt.

Anschließend kann, was mit dem bekannten Messgerät nicht möglich ist, die Leitfähigkeit des Bodens für das Bodenwasser (im folgenden "Bodenwasserleitfähigkeit") ermittelt werden. Die Pumpe saugt zunächst Bodenwasser in die Messkammer, der Boden wird entwässert. Aus dem Wasserfluss, der Wasserspannungsdifferenz/Zeitdauer, der Wasserleitfähigkeit der semipermeablen Membran und der Fließstrecken lässt sich über die Gleichungen nach Darcy und Laplace die Bodenwasserleitfähigkeit messen.

Wählt man gemäß der im Anspruch 7 beschriebenen bevorzugten Ausführungsform eine umsteuerbare Pumpe, so lässt sich anschließend das entnommene Wasser wieder in den Boden zurückdrücken. Dadurch kann die Bodenwasserleitfähigkeit bei Bewässerung (Infiltrationsrate) gemessen werden.

Die Probenahme findet nach der Messung statt. Dazu wir die Schlauchpumpe angesteuert, zum einen für die Gewinnung von definiertem Porenwasser bei einem konstanten Unterdruck, gemessen mit dem Druckaufnehmer und zum anderen für die tensiometergesteuerte Probenahme mit einer zur aktuellen Bodenwasserspannung wählbaren Druckdifferenz.

Mit Hilfe der vor dem Boden des Sammelgefäßes mündenden Förderleitung nach Anspruch 9 lässt sich Bodenwasser zur Bodenoberfläche fördern, so dass das Wasser weiteren Untersuchungen unterzogen werden kann. Die am oberen Ende in das Sammelgefäß mündende Belüftungsleitung dient zum Druckausgleich (Anspruch 10).

Ebenso wie die bekannten misst auch das erfindungsgemäße Tensiometer nur in einem eingeschränkten Messbereich, der durch den Dampfpunkt des Wassers begrenzt (0...ca. 900 hPa) und damit wesentlich kleiner als der pflanzenphysiologisch relevante (0...15.000 hPa) ist.

Das erfindungsgemäße Messgerät ist daher vorzugsweise mit einem Gipsblock versehen oder/und mit einem Psychrometer in Form zweier Thermoelementepaare, die mit dem Boden in Kontakt stehen. Beide sind vorzugsweise in die Gehäuseoberfläche integriert. Mit diesen Zusätzen lässt sich auch der Messbereich oberhalb von etwa 800 hPa abdecken und praktisch bis unendlich messen. Dabei deckt der Gipsblock einen mittleren und das Psychrometer einen oberen Messbereich ab.

Die Genauigkeit des Gipsblocks oder des Psychrometers ist zwar schlecht. Durch die erfindungsgemäße Kombination mit einem Tensiometer lassen sich der Gipsblock und der Psychrometerteil aber kalibrieren, so dass insgesamt mit befriedigender Genauigkeit gemessen werden kann.

Das zur Messung notwendige Wasser wird über die Keramik und die Schlauchpumpe in die Messkammer gesaugt. Befinden sich in der Messkammer Luftblasen, so werden diese vor der Messung mit einem je Messzyklus definierten Volumen, bestehend aus Luft, oder Luftblasen und Wasser oder Wasser über die Peristaltikpumpe in die Steigleitung gefördert. Nachteilig ist: Dadurch wird das Druckgleichgewicht zwischen Bodenwasserspannung und Messkammer gestört. Abhilfe: Die Elektronik regelt die Drehrichtung und Drehgeschwindigkeit der Peristaltikpumpe, bis die Bodenwasserspannung wieder im Gleichgewicht mit dem Druck in der Messkammer steht.

Alternativ dazu kann auch jeweils nach der Messung Luft abgepumpt werden, sofern die Mess-/Pumpintervalle anwenderseitig nicht zu groß gewählt werden. Damit nutzt man die Zeit dazwischen zur Stabilisierung und braucht keine aktive Pumpensteuerung. Dieser Gedanke wird weitergeführt, indem der Befüllvorgang durch zyklisches Pumpen durchgeführt wird.

Insbesondere bei Bodenwasserspannungen zwischen 500 hPa und ca. 900 hPa wird entgastes Wasser benötigt, da in diesem Bereich die Luftblasen bei Messwertänderungen ihr Volumen stark verändern und damit ein größerer Wasseraustausch notwendig wäre, der gerade bei diesen Werten aufgrund der geringeren Bodenwasserleitfähigkeit schlechter möglich ist. Die Entgasung des Tensiometerwassers findet während des Einsaugens des Bodenwassers statt, da die Pumpe einen niedrigeren Druck aufbaut als den des Bodenwassers, wodurch eine Entgasung "in situ" stattfindet.

Ist der Boden bei Werten über dem Dampfpunkt zu trocken, so kann kein Wasser mehr angesaugt werden. Dies wird von der Elektronik detektiert, da sich der Messwert nicht - oder nur unwesentlich verändert, egal, wie die Pumpe läuft. In diesem Fall wird die Bodenwasserspannung mittels des integrierten Gipsblocks oder des Psychrometers gemessen.

Vorzugsweise ist ein Kabel vorgesehen, das von den Anschlüssen der Sensoren und des Steuergeräts an die Bodenoberfläche führt. Legt man dieses Kabel in einen Schlauch, so lässt sich der äußere Luftdruck als Referenzdruck zum Druckaufnehmer führen. Meist reicht aber die Luftdurchlässigkeit von Kabeln in Längsrichtung aus.

Die Erfindung wird anhand des in der Zeichnung gezeigten Ausführungsbeispiels erläutert. Es zeigen:
- Fig. 1: den Längsschnitt des unteren und
- Fig. 2: den Längsschnitt des oberen Teils eines erfindungsgemäßen Messgeräts zur Bestimmung der Wasserspannung in Böden,
- Fig. 3: den Längsschnitt des oberen Teils einer anderen Ausführungsform des erfindungsgemäßen Messgeräts,
- Fig. 4: im Diagramm die Abhängigkeit der Genauigkeit bei Tensiometern und Psychrometern,
- Fig. 5 - 7: im Zeitdiagramm den Betriebsablauf bei verschiedenen Füllzuständen des Messgeräts, und
- Fig. 8: im Zeitdiagramm den Befüllungsvorgang des Messgeräts über zyklisches Pumpen.

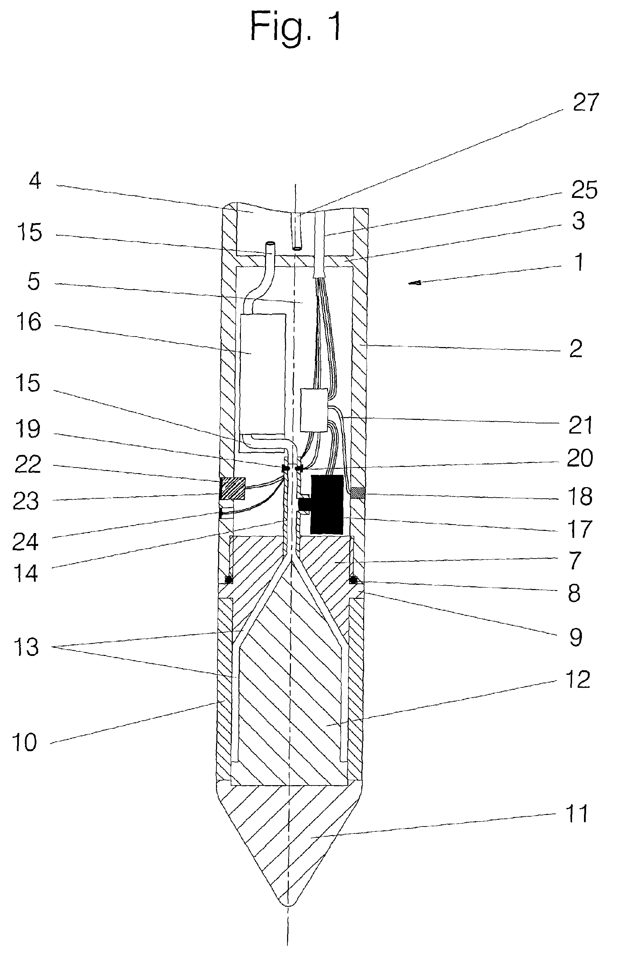

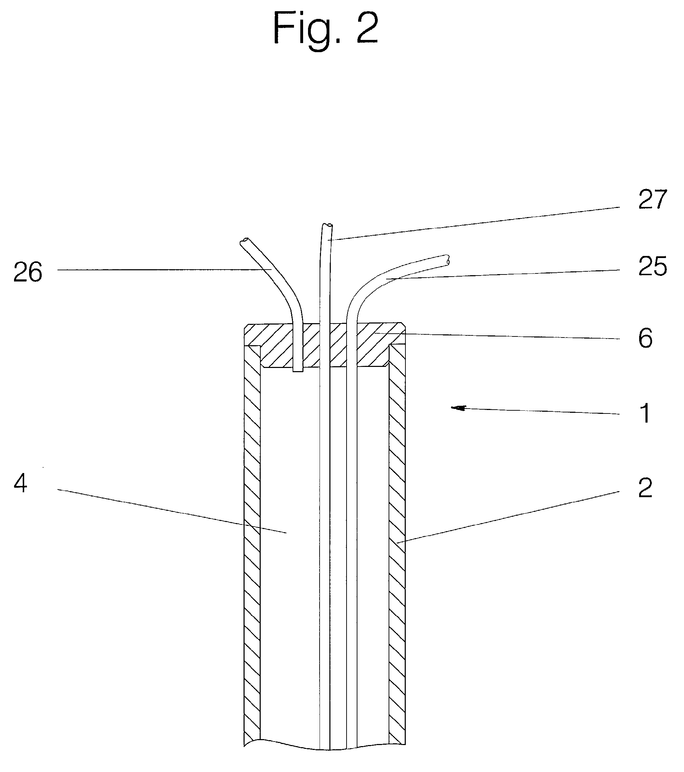

Das in Fig. 1, 2 gezeigte erfindungsgemäße Messgerät 1 besteht aus einem rohrförmigen Gehäuse 2, das durch eine Trennwand 3 in eine obere Kammer 4 und eine untere Kammer 5 unterteilt ist. Die beiden Kammern 4 und 5 sind gegeneinander abgedichtet. Am oberen Ende ist das Rohr 2 durch einen Stopfen 6 verschlossen. In das untere Ende des Rohrs ist ein Träger 7 eingeschraubt, der gegenüber dem Rohr 2 durch einen O-Ring 8 abgedichtet ist. An den Flansch 9 des Trägers 8 ist eine semipermeable Membran in Form einer rohrförmigen Keramik 10 angesetzt, deren unteres Ende durch eine Spitze 11 verschlossen ist. Die Spitze 11 soll das Einbringen des Messgeräts in den Boden erleichtern und dazu beitragen, dass der Boden beim Einbringen möglichst wenig gestört wird. In der vom Träger 7, der Keramik 10 und der Spitze 11 gebildeten Kammer befindet sich ein Füllkörper 12, der diese Kammer praktisch ausfüllt und nur einen kreisrunden, zylindrischen und sich nach oben kegelförmig verjüngenden Strömungskanal 13 freilässt. Der Füllkörper 12 begrenzt das freie Volumen im Einlaufbereich des Messgeräts, damit die in den Boden auslaufende Wassermenge bei Überschreiten des Dampfpunktes minimiert wird. Die Spitze des kegelförmigen Teils des Strömungskanals 13 mündet im Innern einer rohrförmigen Messkammer 14. An die Messkammer 14 schließt eine Steigleitung 15 an, die oberhalb der Trennwand 3 in die obere Kammer 4, die ein Sammelgefäß innerhalb des Messgeräts 1 bildet. In die Steigleitung 15 eingefügt ist eine Schlauchpumpe 16, mit der sich Bodenwasser aus dem Kanal 13 durch Messstrecke 14 in die obere Kammer 4 und in umgekehrter Richtung fördern lässt.

An der Messtrecke 14 sitzt ein Druckaufnehmer 17, der den Druck in der Messstrecke 14 erfasst. An der Messstrecke 14 ist ferner ein Sensor zur Bestimmung des Befüllzustandes angeordnet, der entweder aus einer Infrarotmessstrecke mit einem IR-Sender 19 und einem IR-Empfänger 20 oder aus einem Wärmeflusssensor besteht. Diese erfassen in der Messstrecke 14 befindliche Luftblasen. In der Kammer 5 sitzt ferner eine Steuerelektronik 21. In die Wandung des Gehäuses 2 sind ein Gipsblock 22, der mittels einer Membran 23 gegenüber dem Boden geschützt ist, ein Psychrometer in Form zweier Thermoelementepaare 18 sowie ein Thermofühler 24 integriert, der die Bodentemperatur zu Kompensationszwecken und/oder als auszugebendes Messsignal erfasst.

Mittels eines Schlauches 25 sind die Mess- und Steuerleitungen 26 zum Druckaufnehmer 17, zu den Thermoelementepaaren 18, zum Befüllzustandssensor 19, 20, zur Steuerelektronik 21, zum Gipsblock 22, zum Thermofühler 24 und zur Schlauchpumpe 16 durch den Stopfen 6 und die Trennwand 3 in die untere Kammer 5 geführt. Der Schlauch 25 dient auch zur Zufuhr der Referenzluft an den Druckaufnehmer 17.

Durch den Stopfen 6 hindurch sind schließlich eine Belüftungsleitung 26 und eine Förderleitung 27 in die obere Kammer 4 geführt, wobei die Belüftungsleitung 26 unmittelbar unterhalb des Stopfens und die Förderleitung 27 knapp oberhalb der Trennwand 3 mündet. Die beiden Leitungen 26, 27 sind mittels nicht gezeigter Absperrventile verschließbar.

Die Schlauchpumpe 16 wird von der Steuerelektronik 21 angesteuert und fördert Wasser aus dem Kanal 13 über die Steigleitung 15 in die obere Kammer 4 und umgekehrt. Das gewonnene Bodenwasser kann über die Anzahl der Pumpen-Umdrehungen mengenmäßig begrenzt und über die Förderleitung 27 abgesaugt werden. Über die Belüftungsleitung 26 kann die obere Kammer 4 belüftet oder mit Schutzgas gefüllt werden. Weiter kann durch Anlegen eines Überdruckes das Probenwasser über die Förderleitung 27 herausgedrückt werden.

Die Messelektronik 21 errechnet die Wasserleitfähigkeit, die Wasserspannung bei Bewässerung und Entwässerung sowie den volumetrischen Wassergehalt. Weiter sind über sie die beiden Thermoelementepaare 18 angesteuert. Das Thermoelementepaar 18 wird zur Abkühlung gebracht sowie dessen Temperaturverlauf zur Messung der Wasserspannung ausgewertet. Bei einer Wasserspannung von 500 hPa und 800 hPa wird das Psychrometer von der Messelektronik automatisch auf diese Werte abgeglichen.

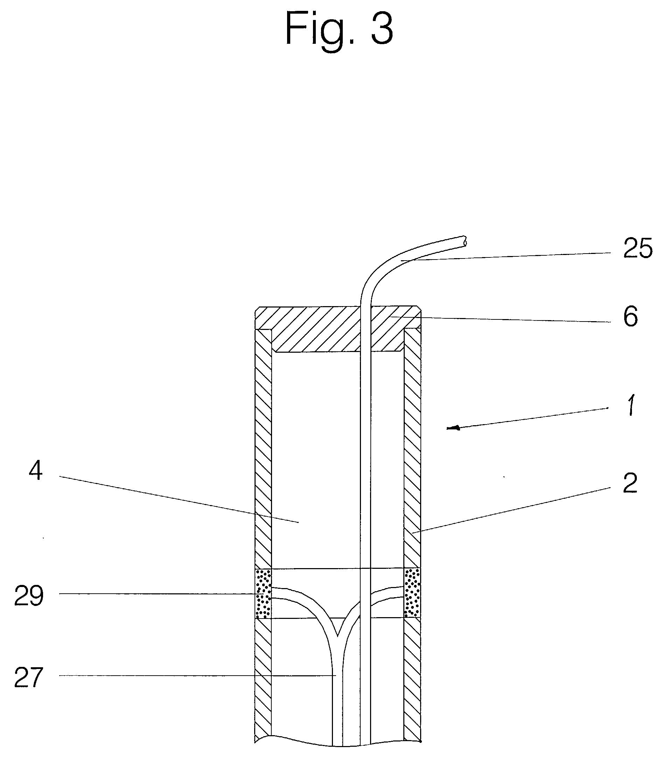

Soll das Bodenwasser nicht an die Oberfläche gefördert, sondern unmittelbar in den Boden rückgeleitet werden, ist gemäß der in Fig. 3 veranschaulichten Ausführungsform die Steigleitung 15 direkt an die Förderleitung 27 angeschlossen. Diese ist ihrerseits an eine kreiszylindrische oder kreissegment-zylindrische Membran 29 angeschlossen. Einzige Verbindung zur "Außenwelt" ist bei dieser Ausführungsform das Kabel 25.

Im Betrieb wird zunächst das Tensiometer in ein Bohrloch eingebaut, das passgenau mit dem Durchmesser der Keramik 1 übereinstimmt. Im Messbetrieb differenziert die Messelektronik drei Fälle:
Fall 1: Messgerät gut befüllt: Der Indikator zeigt "vollständige Befüllung" an.
Fall 2: Messgerät schlecht befüllt: Die Wasserspannung liegt im Bereich 0 ... 800 hPa. - Der Indikator zeigt "Luftblase" an.
Fall 3: Messgerät schlecht befüllt, weil die Wasserspannung höher als 800 hPa liegt.

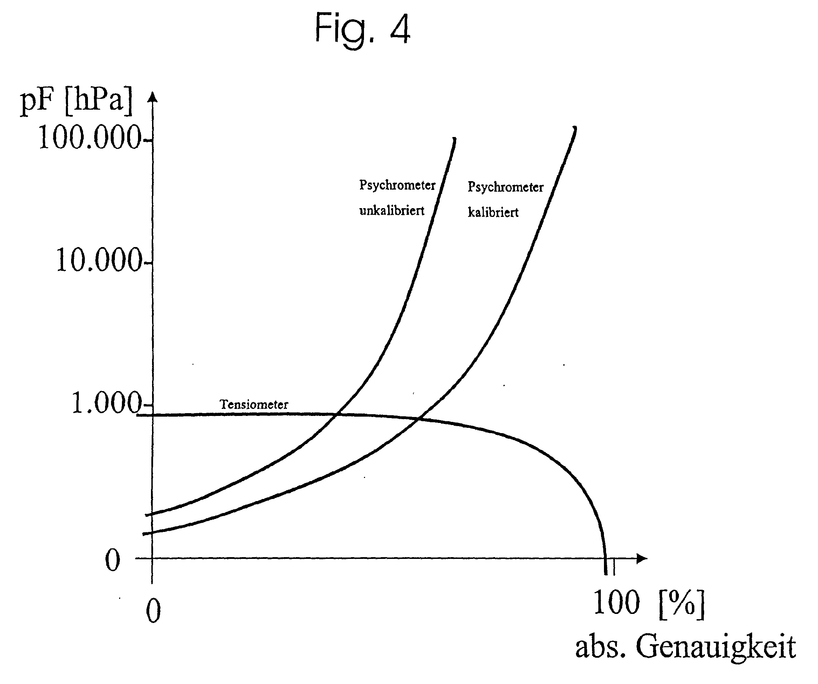

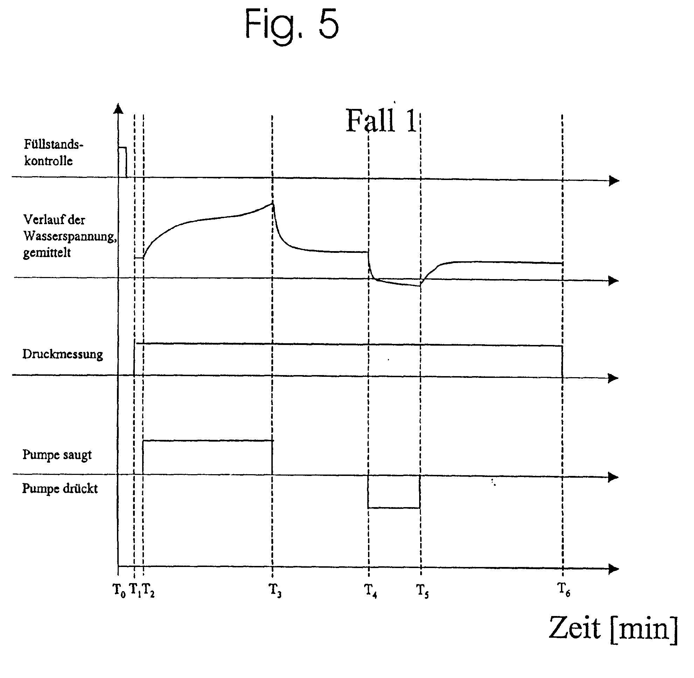

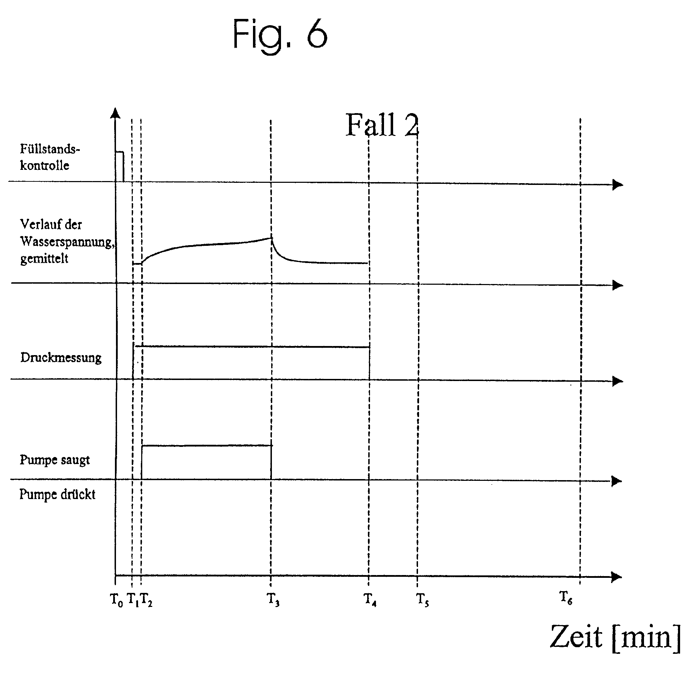

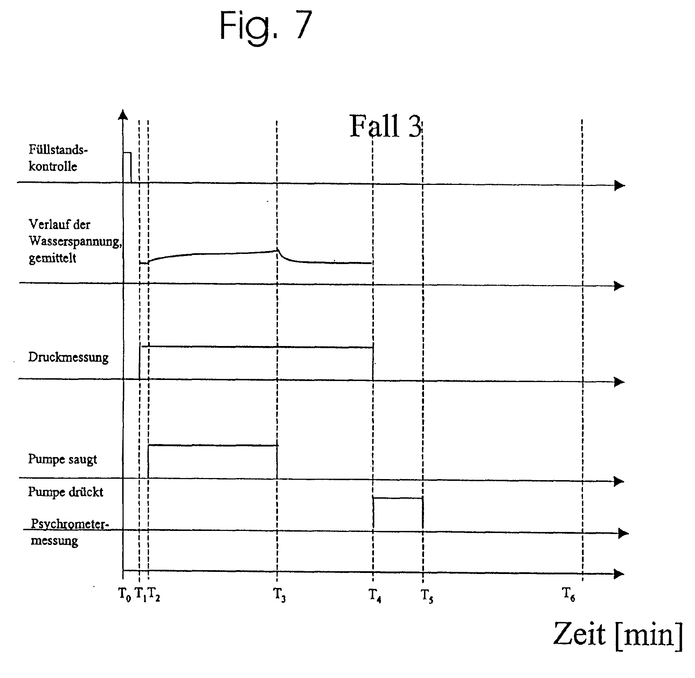

Wie diese Zustände gemessen werden und was für den Messbetrieb daraus resultiert, wird im folgenden anhand von Fig. 4 bis 6 beschrieben:
Fall 1 (Fig. 5): Messgerät gut befüllt, Wasserspannung liegt im Bereich 0 ... 800 hPa:
   Zum Zeitpunkt T₀ wird mit dem Indikator detektiert, ob sich Luftblasen im System befinden. Dies ist unter normalen, feuchten Bedingungen nicht der Fall.
   Zum Zeitpunkt T₁ wird die Wasserspannung gemessen. Liegt diese im Bereich 0 ... 800 hPa, wird sie gespeichert. Zum Zeitpunkt T₂ beginnt die Pumpe Bodenwasser anzusaugen.
   Während der Saugdauer T_{2..3} wird die Wasserspannung gemessen und in Form einiger Mittelwerte gespeichert.
   Zum Zeitpunkt T₃ wird die Pumpe bis T₄ ausgeschaltet, die Wasserspannung gemessen und gespeichert.
   Zum Zeitpunkt T₄ beginnt die Pumpe Wasser zurück in den Boden zu drücken. Während der Drückdauer T_{4..5} wird die Wasserspannung gemessen und gespeichert.
   Zum Zeitpunkt T₅ wird die Pumpe bis T₆ ausgeschaltet und die Wasserspannung gespeichert.
Fall 2 (Fig. 6): Messgerät schlecht befüllt, Wasserspannung liegt im Bereich 0 ... 800 hPa.
   Anmerkung:
   Dies ist insbesondere direkt nach dem Einbau in den Boden der Fall oder wenn die Pumpe 16 längere Zeit nicht angesaugt hat. Ist das Messgerät im Betrieb, so wird die Pumpe derart angesteuert, daß sie stets mehr saugt als Wasser zurück drückt. Dadurch ist im Regelfall das Messgerät gut befüllt (Ausnahme: Der Boden ist trockener als 800 hPa).
   Zum Zeitpunkt T₀ wird mit dem Indikator detektiert, ob sich Luftblasen im System befinden.
   Dies ist hier der Fall.
   Die Pumpe saugt nun so lange, bis der Indikator keine Luftblasen mehr anzeigt oder die Zeit T_{befüll} erreicht wird.
   Während der Saugdauer T₂₋₃ wird die Wasserspannung gespeichert. Anhand der Druckmesswerte wird festgestellt, ob der Messraum 14 durch die Pumpe 12 evakuiert wird oder ob Wasser aus dem Boden in den Messraum 14 fließt.
   Im Anschluss wird für die Zeit T_{3..6} die Pumpe ausgeschaltet, damit sich im Boden wieder ein Wasserspannungsgleichgewicht einstellen kann.
   Zum erneuten Messzeitpunkt beginnt der Indikator wieder mit der Luftblasendetektion (Fall 1 oder nochmals Fall 2).
Fall 3 (Fig. 7): Messgerät schlecht befüllt, weil die Wasserspannung höher als 800 hPa liegt.
   Anmerkung: Dieser Fall tritt ein, wenn der Boden zu trocken ist und deshalb kein Wasser mehr angesaugt werden kann.
   Zum Zeitpunkt T₀ wird mit dem Indikator detektiert, ob sich Luftblasen im System befinden.
   Dies ist hier der Fall.
   Zum Zeitpunkt T₂ beginnt die Pumpe 16 Bodenwasser anzusaugen.
   Während der Saugdauer T₂₋₃ wird die Wasserspannung gespeichert. Anhand der Druckmesswerte wird festgestellt, ob die Messkammer 14 durch die Pumpe 16 evakuiert wird oder ob Wasser aus dem Boden in den Messkammer 14 fließt. Da nun kein Wasser in den Messkammer 14 fließt, wird die Pumpe 16 und die Druckmessung abgeschaltet und das Psychrometer mit den Thermoelementepaaren 18 eingeschaltet.
   Zum erneuten Messzeitpunkt beginnt der Indikator wieder mit der Luftblasendetektion (Fall 1, Fall 2 oder Fall 3).
   Durch Messung des Druckverlaufes können folgende Aussagen gemacht werden:
   Wasserspannung steigt während des Ansaugens schnell:
      -> Die Wasserleitfähigkeit des Bodens ist bei Entwässerung schlecht.
   Im ungesättigten Bereich handelt es sich um einen sandigen oder tonigen Boden. Wasserspannung steigt während des Ansaugens langsam:
      -> Die Wasserleitfähigkeit des Bodens ist bei Entwässerung gut.
   Im ungesättigten Bereich handelt es sich um einen schluffigen Boden. Wasserspannung fällt während des Drückens der Pumpe langsam:
      -> Die Wasserleitfähigkeit des Bodens ist bei Bewässerung schlecht:
         Im ungesättigten Bereich handelt es sich um einen tonigen Boden. Wasserspannung fällt während des Drückens der Pumpe schnell:
      -> Die Wasserleitfähigkeit des Bodens ist bei Bewässerung gut:
         Im ungesättigten Bereich handelt es sich um einen sandigen Boden.

Es kann daher in weiten Bereichen von der Wasserspannung über den k/psi-Zusammenhang bei Bewässerung und Entwässerung auf den Wassergehalt rückgeschlossen werden.

Die Bodenwasserprobenahme funktioniert automatisch dadurch, dass die Pumpe 16 stets etwas mehr saugt als drückt. Ist die gewünschte Wassermenge erreicht, die über die luftblasenfreie Pumpensaugdauer ermittelt wird, wird das Verhältnis luftblasenfreies Saugen/Drücken entsprechend ausgeglichen. Wird das erfindungsgemäße Messgerät trocken, also mit mit Luft gefülltem Strömungskanal 13 in den Boden eingesetzt, kann die Befüllung mit Bodenwasser ergänzend zu Fall 2 (Fig. 6) über zyklisches Pumpen erfolgen. Die Elektronik steuert die Pumpe im Saugbetrieb, bis sich ein vorgegebener Unterdruck im Strömungskanal, gemessen durch den Drucksensor 17, aufbaut. Nach Abschalten der Pumpe gleicht sich der Druck durch die Keramik 10 auf das Niveau der Bodenwasserspannung an. Die Geschwindigkeit, mit der die Anpassung erfolgt, ist abhängig vom Befüllungszustand. Je weniger Luft sich im Strömungskanal 13 befindet, desto schneller erfolgt die Anpassung.

Während der Angleichung der Bodenwasserspannung (die Schlauchpumpe 16 ist ausgeschaltet) wird der sich einstellende Wert durch einige Messpunkte extrapoliert. Die Angleichung der Bodenwasserspannung an den gemessenen Druck beschreibt dabei eine Exponentialfunktion der Form a · e^{b·x} + c. Die Parameter a, b, und c können durch drei zeitlich gewertete Messwerte berechnet werden. Parameter c ist mit der Bodenwasserspannung äquivalent. Die Genauigkeit der Extrapolation nimmt mit zunehmender Befüllung zu.

Bei Erreichen eines zweiten Schwellenwertes des gemessenen Drucks schaltet die Steuerelektronik 21 die Pumpe 16 wieder ein usw.. Die Zeit zwischen diesen Pumpzyklen nimmt mit zunehmender Befüllung ab. Erfasst die Elektronik 21 keine weitere Änderung der Pumpzyklenzeit, so ist der Sensor vollständig gefüllt. Dies kann auch durch den eingebauten Indikator bestätigt werden.

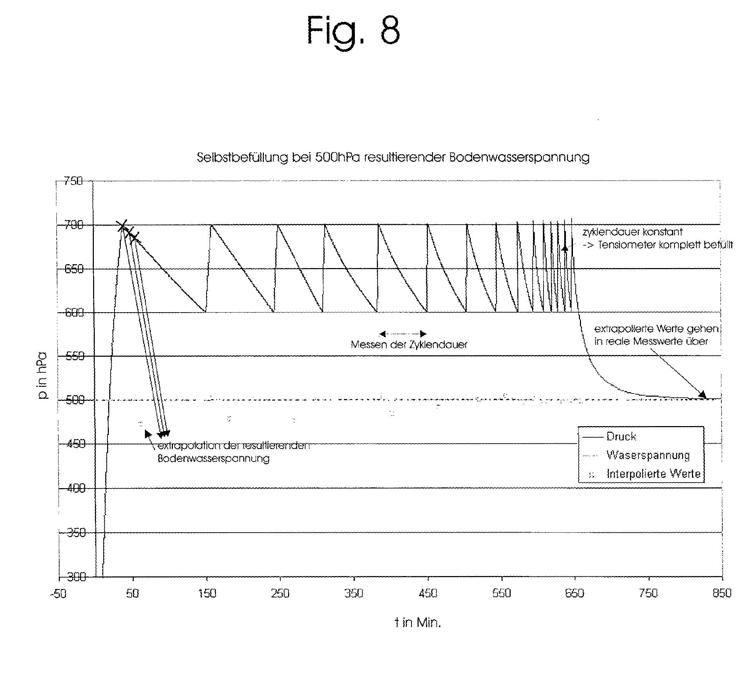

Nach Abbruch der Pumpzyklen gleicht sich nun der innere Druck auf die Bodenwasserspannung an. Der Benutzer des Messgeräts erhält nach Einsetzen desselben bereits nach kurzer Zeit extrapolierte Werte für die Bodenwasserspannung. Nach Beendigung des Befüllens über die Pumpzyklen geht der gelieferte Wert in den real gemessenen über (Fig. 8).

## Patentansprüche

1. Messgerät zur Bestimmung der Bodenwasserspannung mit einem Gehäuse (2) mit einer semipermeablen Membran (Keramik 10), einer innerhalb des Gehäuses (2) angeordneten Messstrecke (14), die über die semipermeable Membran (10) mit dem Bodenwasser in Kontakt steht, **gekennzeichnet durch** eine steuerbare, mit der Messstrecke (14) in Verbindung stehende Pumpe (Schlauchpumpe 16), mittels der Bodenwasser förderbar ist.

2. Messgerät nach Anspruch 1, **gekennzeichnet durch** einen an der Messstrecke (14) angeordneten Druckaufnehmer (17), der an ein Steuergerät (21) angeschlossen ist.

3. Messgerät nach Anspruch 1 oder 2, **gekennzeichnet durch** einen die Gegenwart von Bodenwasser in der Messstrecke erfassenden Sensor (19, 20), der an ein Steuergerät (21) angeschlossen ist.

4. Messgerät nach einem der vorstehenden Ansprüche, **gekennzeichnet durch** eine Steigleitung (15), mittels der das angesammelte Bodenwasser aus dem Gehäuse (2) förderbar ist.

5. Messgerät nach Anspruch 4, **dadurch gekennzeichnet, dass** die Pumpe als direkt auf die Steigleitung (15) wirkende Schlauchpumpe (16) ausgebildet ist.

6. Messgerät nach einem der vorstehenden Ansprüche, **gekennzeichnet durch** ein Sammelgefäß (obere Kammer 4), das an die Steigleitung (15) angeschlossen und vorzugsweise in das Gehäuse (2) integriert ist.

7. Messgerät nach einem der Ansprüche 1 bis 5, **dadurch gekennzeichnet, dass** die Steigleitung (15) an eine in der Gehäusewandung angeordnete Membran (29) angeschlossen ist, über die das Bodenwasser in den Boden rückleitbar ist.

8. Messgerät nach einem der vorstehenden Ansprüche, **dadurch gekennzeichnet, dass** die Pumpe (16) umsteuerbar ist.

9. Messgerät nach einem der vorstehenden Ansprüche, **dadurch gekennzeichnet, dass** die Drehzahl der Pumpe (16) steuerbar ist.

10. Messgerät nach Anspruch 7, **gekennzeichnet durch** eine kurz vor dem Boden des Sammelgefäßes (4) mündende Förderleitung (27).

11. Messgerät nach Anspruch 7, **gekennzeichnet durch** eine an dessen oberem Ende in das Sammelgefäß (4) mündende Belüftungsleitung (26).

12. Messgerät nach einem der vorstehenden Ansprüche, **gekennzeichnet durch** einen mit dem Boden in Kontakt stehenden, vorzugsweise in die Oberfläche des Gehäuses (2) integrierten Gipsblock (22).

13. Messgerät nach einem der vorstehenden Ansprüche, **gekennzeichnet durch** ein mit dem Boden in Kontakt stehendes, vorzugsweise in die Oberfläche des Gehäuses (2) integriertes Psychrometer (Thermoelementepaare 18).

14. Messgerät nach einem der vorstehenden Ansprüche, **gekennzeichnet durch** einen mit dem Boden in Kontakt stehenden, vorzugsweise in die Oberfläche des Gehäuses (2) integrierten Thermofühler (24).

15. Messgerät nach einem der vorstehenden Ansprüche, **dadurch gekennzeichnet, dass** die Anschlüsse der Fühler (17; 18; 19, 20; 22; 23) und des Steuergeräts (21) über ein Kabel (25, 26) aus dem Gehäuse (2) herausgeführt sind, dessen Mantel (25) zur Zufuhr der Referenzluft zum Druckaufnehmer (17) dient.
